# FASCICULE DE BREVET EUROPEEN

(11) **EP 1 044 418 B1**
(45) Date de publication et mention de la délivrance du brevet: **18.06.2003**
(21) Numéro de dépôt: 98964546.0
(22) Date de dépôt: 29.12.1998
(51) Int. Cl.: G06F 17/50

(54) **PROCEDE POUR PREVOIR, IDENTIFIER ET DECRIRE DES MOLECULES SUSCEPTIBLES DE PRESENTER UN COMPORTEMENT RECHERCHE, NOTAMMENT DANS LE DOMAINE DE LA PHARMACIE ET MOLECULES OBTENUES PAR CE PROCEDE**
VERFAHREN ZUR VORHERSAGE, IDENTIFIZIERUNG UND BESCHREIBUNG VON MOLEKÜLEN DIE EIN GEWÜNSCHTES VERHALTEN AUFWEISEN, INBESONDERE IM PHARMAZEUTISCHEN SEKTOR UND DERART HERGESTELLTE MOLEKÜLE
METHOD FOR PROVIDING, IDENTIFYING AND DESCRIBING MOLECULES CAPABLE OF HAVING A REQUIRED ACTIVITY, IN PARTICULAR IN PHARMACOLOGY AND MOLECULES RESULTING FROM SAID METHOD

(30) Priorité: 30.12.1997 FR 9716706
(43) Date de publication de la demande: 18.10.2000
(73) Titulaire: Syntem (S.A.), 300000 Nimes (FR)
(72) Inventeur: GRASSY, Gérard, F-34470 Pérols (FR); KACZOREK, Michel, Synt:Em (S.A.), F-30900 Nîmes (FR); LAHANA, Roger, Synt:Em (S.A.), F-30900 Nîmes (FR); YASRI, Abdelaziz, Synt:Em (S.A.), F-30900 Nîmes (FR)
(74) Mandataire: Hackney, Nigel John
(86) Numéro de dépôt international: FR9802909
(87) Numéro de publication internationale: WO99035598

(56) Documents cités:
- WO-A-97/27559
- GRASSY G ET AL: "Structure-activity relationships of steroids with mineralocorticoid activity" EUROPEAN JOURNAL OF MEDICINAL CHEMISTRY.CHIMICA THERAPEUTICA, vol. 32, no. 11, novembre 1997, page 869-879 XP004100670
- YASRI ET AL: "rational choice of molecular dynamics simulation parameters through the use of the three-dimensional aurocorrelation method : application to calmodulin flexibility study" PROTEIN ENGINEERING., vol. 9, no. 11, novembre 1996, pages 959-976, XP002078077 ENGLAND GB
- GRASSY ET AL: "statistical analysis and shape recognition : applications to md simulations, conformational analysis and structure-activity relationships" TRENDS QSAR MOL. MODELL. PROC.EUR.SYMP. STRUCT.-ACT.RELAT.,1992, pages 216-219, XP002078078

## Description

L'invention concerne la modélisation moléculaire.

Elle a pour objet un nouveau procédé utilisant un certain nombre de molécules de départ, notamment mais non seulement par chimie combinatoire virtuelle, pour prévoir, identifier et décrire des molécules susceptibles de présenter un comportement recherché, notamment dans le domaine de la pharmacie.

On sait que la recherche de nouvelles molécules actives, notamment dans le domaine de la pharmacie, nécessite la synthèse d'un très grand nombre de molécules qu'il faudra ensuite tester in vitro ou in vivo. Dans le meilleur des cas, seul un très petit nombre de ces molécules se révèlera actif.

Pour tenter de rationaliser la recherche de nouvelles molécules actives on a eu l'idée de recourir à la modélisation moléculaire en utilisant des bases de données informatisées. Les résultats obtenus avec les techniques de ce type qui sont connues jusqu'à présent, ne sont pas toujours satisfaisants, en particulier en raison d'une définition insuffisante des paramètres et des critères d'activité.

On connaît également dans l'état de la technique le brevet WO9727559 décrivant un procédé pour sélectionner des molécules par mesure de la distance entre deux descripteurs et par recherche dans une bibliothèque combinatoire virtuelle.

On connaît encore le document YASRI ET AL : « rational choice of molecular dynamics simulation parameters through the use of the three-dimensional aurocorrelation method : application to calmodulin flexibility study » PROTEIN ENGINEERING., vol.9, no. 11, novembre , 1996, pages 959-976 XP002078077 ENGLAND GB divulgue l'utilisation de vecteurs d'autocorrélation tridimensionnelle comme descripteurs, la recherche dans des closses conformotionelles et la comparaison de simulations dynamique de molécules.

La présente invention a pour but de remédier, au moins en partie, à ces inconvénients.

Ce but est atteint selon l'invention qui offre un procédé pour prévoir, identifier et décrire des molécules ayant un comportement recherché, lequel procédé est basé sur la conception assistée par ordinateur et le criblage, également assisté par ordinateur, de banques combinatoires virtuelles. Ce procédé utilise différents descripteurs topologiques, de forme, chimiques, physiques etc. en combinaison avec une nouvelle analyse des trajectoires de dynamique moléculaire. Le terme de "comportement" signifie une "activité" au sens biologique ou pharmacologique, lorsque les molécules concernent des secteurs d'application pharmaceutique, ou une "propriété" au sens physico-chimique, lorsque les molécules concernent des secteurs d'applications non pharmaceutiques, par exemple des matériaux tels que des polymères.

Plus précisément, l'invention a pour objet un procédé assisté par ordinateur pour prévoir, identifier et décrire des molécules ayant un comportement recherché, notamment dans le domaine de la pharmacie, lequel procédé comprend essentiellement les étapes successives consistant à :
1) réaliser une base d'apprentissage à partir d' structures moléculaires voisines connues composées d'une part de structures moléculaires actives connues pour présenter l'activité recherchée et d'autre part de structures moléculaires inactives connues pour être dépourvues de cette activité ou pour présenter une activité recherchée faible, en utilisant des descripteurs appropriés ;
2) générer une série de structures moléculaires, préférentiellement de structures moléculaires voisines, notamment par explosion combinatoire, à partir de la base d'apprentissage ;
3) effectuer un criblage des molécules ainsi générées sur la base de l'enrichissement, en termes de diversité moléculaire, apporté par chaque molécule vis-à-vis des descripteurs choisis ;
4) soumettre les molécules ainsi retenues à des filtres statiques représentatifs des contraintes de variations structurelles, physico-chimiques et moléculaires auxquelles doivent répondre les molécules pour être actives et, éventuellement, synthétiser et tester les molécules sélectionnées ;
5) soumettre les molécules les plus prometteuses ainsi sélectionnées à un filtre dynamique représentatif des contraintes de variation conformationnelle que doivent respecter les molécules pour être actives ;
6) synthétiser et tester les molécules ainsi sélectionnées ;
7) si le résultat escompté n'est pas obtenu, ou s'il n'est obtenu que partiellement, répéter les étapes 3) à 6) en modifiant les filtres.

L'invention concerne également une molécule qui n'était pas antérieurement connue pour présenter un comportement recherché caractérisé en ce qu'elle occupe un espace conformationnel identique ou très voisin de l'espace conformationnel d'une molécule de référence au moins, la molécule de référence étant préalablement connue pour présenter le comportement recherché. La notion de "très voisin" s'apprécie en fonction de l'ensemble des espaces conformationnels correspondant aux molécules testées. Seront par exemple considérés comme "très voisins" les 10% d'espaces conformationnels les plus proches de l'espace conformationnel des molécules de références.

Lorsque le procédé s'appuie sur une pluralité de molécules de références, on considérera comme "très voisins" les espaces conformationnels des molécules de référence.

Pour réaliser une base d'apprentissage, on utilise des descripteurs variés qui peuvent être toutes sortes de propriétés quantitatives et/ou semi-quantitatives. Par exemple, comme cela est connu de l'homme du métier, à partir de la représentation graphique d'une molécule, en d'autres termes une représentation en deux dimensions, ou représentation 2D, d'un composé chimique, on peut déduire un ensemble de valeurs numériques appelées "descripteurs topologiques". On peut en outre utiliser des descripteurs reflétant certaines propriétés physico-chimiques comme par exemple le caractère lipophile, ou lipophilie, qui est exprimé en tant que logP, P étant le coefficient de partition du composé considéré entre l'eau et le n-octanol, ou la réfractivité molaire. On peut en outre utiliser des descripteurs numériques représentant des formes moléculaires.

Les techniques classiquement utilisées dans les corrélations quantitatives de structure-activité (Quantitative Structure Activity Relationships : QSAR) sont basées sur l'hypothèse que si une molécule présente un comportement biologique donné, toutes les informations nécessaires pour la caractériser se fondent fortement sur sa structure, c'est-à-dire ses atomes, ses liaisons et ses formes. Contrairement au paradigme QSAR linéaire classique dans lequel l'activité biologique peut être exprimée sous la forme d'une combinaison linéaire de descripteurs pertinents, la Demanderesse utilise un paradigme de représentation d'informations ou mappage variable, non linéaire, dans lequel l'activité est une fonction non linéaire de descripteurs structuraux, topologiques et moléculaires.

Selon l'invention, on analyse un ensemble de descripteurs pour les composés actifs, en opposition aux composés inactifs, ce qui permet la définition de filtres pour faire la différence entre les deux classes de molécules [G. Grassy & al., J. Mol. Graphics, vol. 13, page 356 (1995)].

Si l'échantillon d'apprentissage correspond à des tests *in vivo*, le jeu de filtres décrit les conditions d'activité *in vivo*. Dans le cas des tests *in vitro*, le jeu de filtres décrit les conditions d'activité *in vitro*.

Un filtre est défini par la plage de variation d'un descripteur donné pour tous les composés actifs connus lorsqu'on la compare à la plage de variation du même descripteur pour tous les composés inactifs connus.

Si la plage "active" recouvre totalement la plage "inactive", le filtre ne sert à rien, ce qui revient à dire que la variation de ce descripteur particulier n'a pas de relation avec l'activité biologique.

Si la plage "active" ne recouvre pas totalement la plage "inactive", toute molécule présentant pour ce descripteur une valeur qui tombe dans une zone de la plage "active" qui ne recouvre pas la zone "inactive" aura une forte probabilité d'être active aussi.

Les aptitudes à la prédiction de ce type d'approche sont améliorées par l'utilisation de plusieurs filtres, préférentiellement 10 à 30, chacun représentant une manière différente de quantifier les propriétés structurelles et physico-chimiques d'une molécule.

Ces filtres, une fois établis, peuvent être utilisés pour cribler des composés virtuels générés pendant une explosion combinatoire, c'est-à-dire tous les composés obtenus par exemple en branchant une liste de substituants sur des parties prédéterminées d'un noyau structurel appelé synthon. Les composés les plus prometteurs, à savoir ceux qui satisfont à toutes les contraintes définies par les filtres, sont ensuite synthétisés par synthèse chimique ou par génie génétique, ou encore par tout autre moyen, et testés dans des essais biologiques.

Les molécules inactives que l'on avait prédites actives contiennent des caractéristiques inexplorées qui sont déterminantes pour l'activité. De telles propriétés sont utilisées pour la définition de filtres additionnels et, après quelques répétitions, le processus de criblage virtuel peut être utilisé pour l'identification exacte de composés présentant les propriétés recherchées.

En pratique, à partir des structures de départ dont l'activité peut être mesurée *in vitro* et/ou *in vivo*, on réalise une "base d'apprentissage" en déterminant les différents paramètres physico-chimiques, structuraux et moléculaires permettant de décrire les molécules actives et les molécules inactives.

Ces paramètres sont représentés sous forme de plages de variations, ou "contraintes", en fonction des classes d'activité. Un jeu ou ensemble de contraintes définit un filtre.

La qualité des descripteurs choisis est contrôlée en termes de variabilité, ainsi que le choix des descripteurs à utiliser et l'intercorrélation entre les descripteurs retenus.

Ceci peut être réalisé en utilisant un logiciel conçu par R. Lahana, le logiciel ANODA (ANalysis Of DAta) qui associe à une chimiothèque une "carte de visite" simple en termes de descripteurs de base et d'analyse en composantes principales. Différentes techniques de sélection des descripteurs telles qu'analyses de variabilité, d'intercorrélation, de représentativité (régressions, réseaux de neurones, algorithmes génétiques) sont mises en oeuvre.

Une explosion combinatoire est générée à partir de la base d'apprentissage et en prenant en compte les conditions de variation utiles pour chaque position variable. Pour ce faire, il est possible d'utiliser des bases de données de substituants définis par leur structure et les descripteurs qui en découlent.

L'explosion combinatoire peut être générée en utilisant du logiciel LEGION de la société TRIPOS ou un autre logiciel conçu par R. Lahana, le logiciel COMBEX qui crée une telle explosion à partir d'un répartiteur ("scaffold") ou d'une séquence consensus, et d'une liste de contraintes sur chacun des points de substitution. Un langage de commande de type SQL (Standard Query Language) permet de combiner à volonté toutes sortes de conditions de sélection des substituants.

Pour chaque combinaison générée, il est possible de vérifier l'enrichissement en terme de diversité moléculaire apportée par la molécule créée vis-à-vis des descripteurs choisis. Si cet enrichissement est suffisant, la molécule est conservée, sinon elle est rejetée.

L'enrichissement en terme de diversité moléculaire peut être vérifié au moyen d'un logiciel conçu par R. Lahana, le logiciel DIVERSER qui permet d'effectuer une évaluation quantitative de la diversité moléculaire présentée par une chimiothèque quelconque, même si les molécules qui la composent sont hautement flexibles et si cette chimiothèque est purement virtuelle. Ce logiciel permet de comparer des banques en terme de diversité, de caractériser des "trous" de diversité dans une banque, de concevoir rationnellement des banques minimales ayant une diversité maximale. Les techniques de sélection peuvent être par exemple la classification hiérarchique (clusters), des partitions, la conception uniforme (uniform design), le tirage aléatoire, etc.

Les filtres "statiques", c'est-à-dire ceux qui ne dépendent pas de la variabilité conformationnelle des molécules, décrivent les intervalles des valeurs structurales, physico-chimiques, moléculaires, auxquels les molécules doivent correspondre afin d'être actives. En utilisant ces filtres statiques, on déduit les molécules ayant le plus de chances d'être actives. Ces molécules sont éventuellement synthétisées et testées pour l'activité recherchée.

Dans cette étape, on peut utiliser un logiciel conçu par G. Grassy, le logiciel VARIMAP qui établit des filtres statiques à partir de descripteurs obtenus par TSAR qui est un logiciel commercialisé par Oxford Molecular Group Oxford (Royaume-Uni) et dont les auteurs initiaux sont G. Grassy et R. Lahana.

Les candidats les plus prometteurs sélectionnés au moyen des filtres statiques sont ensuite soumis à un filtre dynamique, c'est-à-dire représentatif des contraintes de variation conformationnelle que doivent respecter les molécules générées pour avoir des chances d'être actives, par comparaison avec celles de la base d'apprentissage.

Ce filtrage dynamique peut être effectuée au moyen d'un logiciel nouvellement conçu par G. Grassy, le logiciel MULTIDYN qui permet, à partir de trajectoires de dynamique moléculaire, de caractériser les espaces conformationnels de molécules quelconques. Les conformations bioactives des molécules en question sont ainsi mises en évidence.

Les molécules retenues sont ensuite synthétisées et testées.

Si l'activité biologique trouvée ne répond pas ou ne répond que partiellement aux critères attendus, les étapes de vérification de l'enrichissement en terme de diversité moléculaire, de filtrage statique, de filtrage dynamique, de synthèse et d'essais sont répétées.

Si on trouve que des molécules répondent aux critères biologiques fixés, le but est atteint.

Le procédé selon l'invention permet donc de définir de nouvelles molécules actives par un processus entièrement rationel n'utilisant à aucun moment d'hypothèse sur leur mécanisme d'action possible ou sur leur récepteur éventuel.

Le procédé selon l'invention a été mis en oeuvre avec succès dans des domaines de pharmaco-modulation de molécules afin d'améliorer les performances de ces molécules *in vitro* et surtout *in vivo*.

L'exemple détaillé de mise en oeuvre qui suit sert à illustrer et mieux expliquer l'invention.

### EXEMPLE

### Obtention de nouveaux composés immuno-modulateurs

On a récemment montré que le peptide 2702.75-84 (peptide dérivé du HLA-B2702, acides aminés 75 à 84) prolonge, chez la souris, la survie après une allo-greffe du coeur.(Transplantation, vol. 59, page 455 (1995).

L'administration du peptide 2702.75-84 à raison de 80 mg/kg/jour pendant 10 jours après la transplantation de coeurs B6 chez des receveurs CBA prolonge la survie à l'allo-greffe de coeur jusqu'à 11,4 2,6 (n=8) jours comparés à 8,2 1,2 jours chez les animaux témoins non traités (p<0,01). Aucun effet sur la survie à la greffe n'a été observé après une thérapie avec le peptide 2702.75-84 à des doses inférieures.

L'activité *in vivo* de plusieurs peptides (n=19) dérivés du peptide 2702.75-84 et d'autres séquences MHC/HLA a été évaluée de façon similaire. Les peptides testés comprenaient des peptides de types acides aminés D et L. Certains peptides différaient du peptide 2702.75-84 sur jusqu'à six positions d'acides aminés, d'autres avaient une séquence inversée.

Tous ces peptides dont les séquences sont représentées dans le tableau I qui suit, ont été synthétisés en utilisant la chimie F-moc/tBu puis testés.

Les études de réponse aux doses sur des souris ayant reçu une allo-greffe de coeur ont été effectuées en utilisant des peptides sous la forme acétate, purifiés à plus de 90% par chromatographie liquide haute performance (HPLC).

La transplantation cardiaque hétérotrope abdominale a été effectuée comme décrit précédemment J. Thorac. Cardiovasc. Surg., vol. 7, page 225 (1969).

Des souris CBA receveuses de coeurs C57B1/6 ont été traitées quotidiennement avec différentes doses de peptide après la transplantation d'organe. Les peptides étaient dissous dans du DMSO et dilués dans du PBS (concentration finale en DMSO 10%) avant l'administration intrapéritonéale. Les animaux ont été traités à partir du jour de la transplantation jusqu'au 9ème jour. La survie à la greffe a été surveillée quotidiennement par palpation directe et le rejet a été défini comme la fin de la contractilité cardiaque palpable. La significance statistique de la prolongation de survie à l'allo-greffe du coeur a été calculée en utilisant le test de Mann-Whitney.

La thérapie des receveurs de coeur de souris allo-greffe avec certains de ces peptides (n=9 ; en gras dans le tableau I qui suit) a permis une prolongation significative de la survie à la greffe, alors que les autres n'avaient pas d'effets significatifs.

Les structures des peptides utilisés et les résultats obtenus sont présentés dans le tableau I qui suit.

**TABLEAU I**

| Séquence peptidique | HLA/MHC | MSTSD |
|---|---|---|
| Non traité | | 7,5 1,1 |
| RENLRIALRY | B2702 | 11,4 2,6 |
| YRLAIRLNER | - | 12,1 2,8 |
| renlrialry | - | 11,4 4,1 |
| yrlairlner | - | 13,2 2,7 |
| RVNLRIALRY | - | 11,5 0,5 |
| YRLAIRLNVR | - | 12,5 1,6 |
| rvnlrialry | - | 13,1 3,9 |
| yrlairlnvr | - | 12,2 2,9 |
| NLRIALRYYW | - | 11,6 1,3 |
| RVNLRTALRY | Kk | 8,5 0,7 |
| RVDLRTLLRY | Dk | 7,0 0,5 |
| RVDKRTLLGY | Kb | 7,8 1,0 |
| RVSLRNLLGY | Db | 8,0 0,5 |
| RESLRLLRGY | 07 | 7,5 0,7 |
| REDLRTLLRY | B2705 | 7,7 1,2 |
| ENLRIALR | - | 8,5 0,7 |
| renlpialry | - | 9,5 2,4 |
| RVNLRTLRRY | E | 8,0 0,5 |
| RMNLQTLRGY | G | 7,5 0,7 |

L'ensemble des 19 peptides a été utilisé comme base d'apprentissage pour définir la stratégie de conception rationelle.

Bien que cet ensemble de départ soit très petit, il a permis, comme le démontre la distinction initiale entre les composés actifs et les composés inactifs, une définition efficace des contraintes.

Initialement, 27 descripteurs indépendants de la conformation ont été calculés pour chaque peptide (voir le Tableau II ci-après).

Les descripteurs physico-chimiques et topologiques ont été générés par le logiciel TSAR V2.31 (Oxford Molecular Group, Oxford, Royaume-Uni).

Ces 27 descripteurs comprenaient le moment dipolaire, calculé sur la base d'une conformation totalement développée de chaque peptide. L'analyse statistique a montré que 14 descripteurs étaient intercorrélés. Par conséquent, ils n'étaient pas utiles pour la définition de contraintes qui différencient entre les peptides actifs et les inactifs. Les 13 autres descripteurs, indépendants de la conformation, ont été utilisés pour construire un filtre statique pour le criblage d'une bibliothèque combinatoire virtuelle comme décrit plus loin.

**TABLEAU II**

| Les 27 descripteurs topologiques et physicochimiques utilisés | | |
|---|---|---|
| Propriété | Nature | Inclus/exclu |
| *Masse molaire* | *Physique* | *Exclu* |
| Volume ellipsoïdal | Forme | Inclus |
| Volume moléculaire | Forme | Inclus |
| Réfractivité molaire | Topologique | Inclus |
| Lipophilie (LogP) | Topologique | Inclus |
| *Kappa 1* | *Topologique* | *Exclu* |
| *Kappa 2* | *Topologique* | *Exclu* |
| *Kappa 3* | *Topologique* | *Exclu* |
| *Kappa Alpha 1* | *Topologique* | *Exclu* |
| Kappa Alpha 2 | Topologique | Inclus |
| *Kappa Alpha 3* | *Topologique* | *Exclu* |
| Flexibilité | Topologique | Inclus |
| Kier Chi V4 | Topologique | Inclus |
| *Indice Randic* | *Topologique* | *Exclu* |
| Indice Balaban | Topologique | Inclus |
| *Indice Wiener* | *Topologique* | *Exclu* |
| *Somme état E* | *Physique* | *Exclu* |
| Moment dipolaire | Physique | Inclus |
| *Nombre d'atomes de C* | *Chimique* | *Exclu* |
| Nombre d'atomes de O | Chimique | Inclus |
| Nombre d'atomes de N | Chimique | Inclus |
| *Nombre d'atomes de H* | *Chimique* | *Exclu* |
| *Nombre total d'atomes* | *Chimique* | *Exclu* |
| *Nombre de groupes méthyle* | *Chimique* | *Exclu* |
| Nombre de groupes éthyle | Chimique | Inclus |
| *Nombre de groupes amino* | *Chimique* | *Exclu* |
| Nombre de groupes hydroxy | Chimique | Inclus |

Les descripteurs topologiques listés ci-dessus ont été utilisés pour calculer les filtres statiques. Treize descripteurs (en écriture droite), ont fourni des informations sur les caractéristiques des peptides ayant une activité immuno-modulatrice et ont été utilisés pour définir des contraintes pour cribler une bibliothèque combinatoire virtuelle. Les quatorze autres descripteurs se sont révélés être intercorrélés et ont été exclus de l'analyse.

En utilisant le programme COMBEX (Synt:em, Nîmes, France), on a généré une explosion combinatoire basée sur une séquence consensus RXXXRXXXXY, dérivée de l'ensemble d'apprentissage, après alignement de toutes les séquences actives et inactives. Cette séquence a laissé 7 positions, les positions représentées par "X", pour effectuer des mutations afin de créer la bibliothèque.

Toutes les molécules ont été générées en utilisant la convention SMILES puis converties en une structure 3D en utilisant le logiciel CORINA (Oxford Molecular Group, Oxford, Royaume-Uni).

Initialement, des acides aminés tant naturels que non naturels étaient compris dans la base de données de substituants. Tous les acides aminés étaient décrits en termes de propriétés physico-chimiques (lipophilie, basicité, aromaticité, etc.) et également par des descripteurs topologiques (analyse de Kier, indice Balaban, etc.). En utilisant 35 acides aminés, cela conduisait à 357 combinaisons, soit 64 milliards de composés, ce qui était encore trop important pour la capacité de l'ordinateur utilisé.

Afin de diminuer ce nombre, quelques données supplémentaires telles que la distribution de la lipophilie, ont été prises en compte. En fait, la structure du composé "conducteur" (2702.75-84) présentait deux domaines lipophiles séparés par des résidus hydrophiles. Ceci suggérait qu'une telle distribution était importante pour l'activité. Afin de respecter cette distribution, la Demanderesse a décidé d'utiliser, pour chacune des 7 positions, la liste suivante d'acides aminés : V, I, T, W, L, nL (nL = norleucine). Ainsi, le nombre des composés de la bibliothèque a été ramené à 67 combinaisons, soit 279 936 composés.

Pour cribler la bibliothèque combinatoire virtuelle, on a calculé "au vol", pour chaque structure générée par le logiciel COMBEX, l'ensemble correspondant de propriétés. Ces propriétés ont été analysées en utilisant les filtres statiques et les filtres dynamiques prédéfinis, basés sur les contraintes et on n'a retenu que les composés satisfaisant à toutes les contraintes.

Les filtres statiques, obtenus par les plages de variation des descripteurs retenus étaient basés sur l'ensemble de contraintes indépendantes de la conformation défini en utilisant l'ensemble d'apprentissage de composés actifs et inactifs. Ils ont été conçus en utilisant le logiciel VARIMAP (Synt:em, Nîmes, France).

En criblant la bibliothèque de 279 936 composés avec ces filtres statiques, on a identifié 26 peptides qui satisfaisaient à toutes les contraintes. Parmi ceux-ci les peptides ont été étudiés du point de vue de leurs espaces conformationnels (filtre dynamique) et en particulier 5, appelés respectivement RDP1257, RDP1258, RDP1259, RDP1271 et RDP1277 ont été étudiés.

Le caractère flexible des peptides a été analysé en utilisant des simulations de dynamique moléculaire (MD).

Les simulations MD des peptides solvatés avec des conditions périodiques ont été effectuées en utilisant le logiciel AMBER 4.1 (Oxford Molecular Group, Oxford, Royaume-Uni). Il utilisait une durée de 1015 ps pour chaque peptide solvaté. La constante diélectrique été réglée sur l'unité. La température du système a été initialement portée progressivement de 10 à 300 K, sur une période de 15 ps. Pendant la simulation, on a maintenu une température constante de 300 ± 10 K en couplant à un bain extérieur avec une durée de relaxation de 0,1 ps. Le "pas" temporel choisi était de 1 fs. Le temps de calcul était approximativement de 0,5 h par ps. On a utilisé une coupure "basée sur les résidus" de 10 Å pour toutes les interactions non liées. La liste des paires non liées était remise à jour toutes les 10 fs et les coordonnées étaient recueillies chaque ps pendant les trajectoires, ce qui a donné un ensemble de 1015 conformations pour chaque trajectoire. Dans toutes les trajectoires, aucune contrainte n'a été appliquée aux atomes et aucun "terme parasite" n'a été utilisé dans l'expression d'énergie.

Chaque conformation a été représentée par un descripteur de forme appelé le vecteur d'autocorrélation 3D ci-après "3D-ACV" [Eur. J. Med. Chem.- Chim. Theor., vol. 19, page 61 (1984)]. Un ensemble de 3D-ACV a été calculé pour chaque série de conformations obtenues par dynamique moléculaire (MD) puis traité en utilisant les statistiques à plusieurs variables [Trends in QSAR and Molecular modelling 92 ESCOM Publishers, page 216 (1993)]. Ceci a été réalisé en trois étapes principales (i à iii):
(i) Pour une conformation donnée de la molécule étudiée, ici un peptide, le descripteur 3D-ACV correspondant a été calculé comme suit. Les distances entre toutes les paires d'atomes ont été calculées. La distribution de ces distances était un vecteur dont chaque "boîte" (bin) était la somme de paires d'atomes dans une gamme spécifique de séparation interatomique, c'est-à-dire où deux atomes donnés étaient séparés par une distance comprise entre (r-1) et r Å. Dans ce travail, le pas était égal à 1 Å. Évidemment, la moindre modification de la conformation de ladite molécule aboutit à un changement de la distribution des distances interatomiques, donc à une modification du 3D-ACV. Par conséquent, ce descripteur est l'un des plus efficaces pour représenter les formes conformationnelles des molécules.
(ii) Une trajectoire MD est l'ensemble des conformations adoptées par une molécule donnée pendant la simulation MD. Pour chaque conformation, le 3D-ACV correspondant a été calculé "au vol" et mémorisé. Ce 3D-ACV multiple, fonction du temps est un descripteur du comportement dynamique de ladite molécule.
(iii) Pour la comparaison des 3D-ACV multiples représentant les trajectoires de l'ensemble de molécules analysé, on a appliqué une analyse de composants principaux (PCA) à chacun de ces 3D-ACV multiples. Cette transformation a réduit les dimensions de l'ensemble de données à un nombre plus faible (dans le cas présent, un espace 2D) et a aussi permis la projection de toutes les trajectoires de toutes les molécules sur un plan. Dans cet espace réduit, chaque molécule est représentée par un ensemble de points, c'est-à-dire ses conformations pendant toute la durée de la simulation MD, ce qui représente son espace conformationnel. Les molécules pouvaient ensuite être comparées l'une à l'autre en termes d'espaces conformationnels. Les analyses des trajectoires et des espaces conformationnels ont été calculés au moyen du logiciel MULTIDYN (Synt:em, Nîmes, France).

Par utilisation d'un filtre dynamique pour cribler les peptides en termes d'espaces conformationnels, il est apparu que 4 de ces 5 peptides occupaient le même espace conformationnel ou un espace très similaire mais que l'un, le RDP1277, était différent de ce point de vue.

Ces 5 peptides ont été synthétisés et testés dans des essais biologiques de survie à l'allo-greffe du coeur.

A une dose de 10 mg/kg/jour, tous ces peptides, sauf le RDP1277, étaient actifs *in vivo*. Aucune prolongation significative de survie à la greffe n'a été observée après la thérapie avec le RDP1277 (MST = 9,0 1,4). En revanche, la thérapie avec tous les autres peptides a permis une prolongation significative de la survie à la greffe, allant de 11 à 13 jours,

Des essais supplémentaires avec le peptide RDP1258 à des doses de 1 à 10 mg/kg/jour ont révélé une efficacité accrue de la thérapie aux doses inférieures. A une dose de 1 mg/kg/jour, 30% des souris ayant reçu une allo-greffe de coeur et traitées par le RDP1258 on survécu pendant plus de 100 jours, alors que le peptide de référence, 2705.75-84, prolongeait la survie à l'allo-greffe de coeur après une thérapie à raison de 80 mg/kg/jour, tandis qu'aucun effet n'était observé aux doses inférieures. De plus, aucune survie de longue durée à la greffe n'a été induite par une thérapie utilisant ce peptide de référence.

La stratégie selon l'invention a donc bien permis de concevoir de façon rationnelle plusieurs composés bioactifs dont l'activité s'est révélée supérieure à celle des molécules actives de la base d'apprentissage.

## Revendications

1. Procédé assisté par ordinateur pour prévoir, identifier et décrire des molécules ayant un comportement recherché, notamment dans le domaine de la pharmacie, **caractérisé en ce qu'**il comprend:
1) prévoir une base d'apprentissage à partir d'une famille de structures moléculaires connues composée d'une part de structures moléculaires actives présentant l'activité recherchée et d'autre part de structures moléculaires inactives connues dépourvues de cette activité, ou comme présentant une activité faible, en utilisant des descripteurs appropriés;
2) générer des filtres statiques représentatifs des intervalles des valeurs structurelles, physico-chimiques et moléculaires auxquels doivent répondre les molécules pour être actives à partir de la base d'apprentissage;
3) effectuer un criblage d'une banque combinatoire par suite de ces filtres statiques;
4) soumettre les molécules les plus prometteuses ainsi sélectionnées à un filtre dynamique représentatif des contraintes de variation conformationnelle que doivent respecter les molécules pour être actives.

2. Procédé assisté par ordinateur pour prévoir, identifier et décrire des molécules ayant un comportement recherché, notamment dans le domaine de la pharmacie, selon la revendication 1, **caractérisé en ce qu'**il comprend essentiellement les étapes successives consistant à:
1) réaliser une base d'apprentissage à partir d'une famille de structures moléculaires connues composée d'une part de structures moléculaires actives présentant l'activité recherchée et d'autre part de structures moléculaires inactives connues dépourvues de cette activité, ou comme présentant une activité faible, en utilisant des descripteurs appropriés;
2) générer une explosion combinatoire de molécules à partir de la base d'apprentissage;
3) effecteur un criblage des molécules ainsi générées sur la base de l'enrichissement, en termes de diversité moléculaire, apporté par chaque molécule vis-à-vis des descripteurs choisis;
4) soumettre les molécules ainsi retenues à des filtres statiques représentatifs des contraintes de variations structurelles, physico-chimiques et moléculaires auxquelles doivent répondre les molécules pour être actives et, éventuellement, synthétiser et tester les molécules sélectionnées ;
5) soumettre les molécules les plus prometteuses ainsi sélectionnées à un filtre dynamique représentatif des contraintes de variation conformationnelle que doivent respecter les molécules pour être actives;
6) synthétiser et tester les molécules ainsi sélectionnées.

3. Procédé assisté par ordinateur pour prévoir, identifier et décrire des molécules ayant un comportement recherché, notamment dans le domaine de la pharmacie, selon la revendication 2, **caractérisé en ce que**, si le résultat escompté n'est pas obtenu, ou s'il n'est obtenu que partiellement, les étapes 3) à 6) sont répétées en modifiant les filtres.

4. Procédé assisté par ordinateur pour prévoir, identifier et décrire des molécules ayant un comportement recherché, notamment dans le domaine de la pharmacie, selon la revendication 2 ou 3, **caractérisé en ce que** le filtrage dynamique peut être effectuée au moyen d'un logiciel qui permet, caractérisé les espaces conformationnels de molécules quelconques à partir de trajectoires de dynamique moléculaire.

5. Procédé assisté par ordinateur pour prévoir, identifier et décrire des molécules ayant un comportement recherché, notamment dans le domaine de la pharmacie, selon l'une quelconque des revendications précédentes, **caractérisé en ce que**, le filtrage dynamique met en oeuvre le descripteur de forme constitué par le vecteur d'autocorrélation 3D (3D-ACV).

6. Procédé assisté par ordinateur pour prévoir, identifier et décrire des molécules ayant un comportement recherche, notamment dans le domaine de la pharmacie, selon la revendication 5, **caractérisé en ce que** le filtrage dynamique consiste à calculer un ensemble de 3D-ACV pour chaque série de conformations obtenues par dynamique moléculaire (MD) puis traité en utilisant les statistiques à plusieurs variables en trois étapes principales (i à iii):
(i) Pour une conformation donnée de la molécule étudiée, on calcule le descripteur 3D-ACV correspondant par détermination des distances entre toutes les paires d'atomes et de la distribution de ces distances formant un vecteur dont chaque "boîte" (bin) est la somme de paires d'atomes dans une gamme spécifique de séparation interatomique;
(ii) On calcule on mémorise pour chaque conformation, le 3D-ACV multiple correspondant "au vol";
iii) On procède à une analyse de composants principaux (PCA) à chacun de ces 3C-ACV multiples.

7. Procédé assisté par ordinateur pour prévoir, identifier et décrire des molécules ayant un comportement recherché, notamment ans le domaine de la pharmacie, selon la revendication 4, **caractérisé en ce que** le filtre statique met en oeuvre des descripteurs physico-chimiques et topologiques dont une partie au moins est choisie parmi masse molaire, volume ellipsoïdal, volume moléculaire, réfractivité molaire, lipophilie (LogP), kappa 1, kappa 2, kappa 3, kappa alpha 1, kappa alpha 2, kappa alpha 3, flexibilité, kier chi V4, indice randic, indice balaban, indice wiener, somme état E, moment dipolaire, nombre d'atomes de C, nombre d'atomes de O, nombre d'atomes de N, nombre d'atomes de H, nombre total d'atomes, nombre de groupes méthyle, nombre de groupes éthyle, nombre de groupes amino et nombre de groupes hydroxy.

## Patentansprüche

1. Computergestütztes Verfahren zum Bereitstellen, Identifizieren und Beschreiben von Molekülen mit einem gewünschten Verhalten, insbesondere auf dem Gebiet der Pharmakologie, **dadurch gekennzeichnet, dass** es Folgendes umfasst:
1) das Bereitstellen einer Lernbasis, ausgehend von einer Gruppe bekannter Molekülstrukturen, die einerseits aus aktiven Molekülstrukturen, die die gewünschte Aktivität aufweisen, und andererseits aus bekannten inaktiven Molekülstrukturen besteht, denen diese Aktivität fehlt oder die schwache Aktivität aufweisen, indem entsprechende Deskriptoren eingesetzt werden;
2) das von der Lernbasis ausgehende Schaffen statischer Filter, die für die Intervalle der Strukturwerte, der physiochemischen und der molekularen Werte repräsentativ sind, denen die Moleküle entsprechen müssen, um aktiv zu sein;
3) das von diesen statischen Filtern ausgehende Durchführen einer Sichtung einer kombinatorischen Bank;
4) das Filtern der vielversprechendsten so selektierten Moleküle mit einem dynamischen Filter, der für Beschränkungen der Konformationsschwankung repräsentativ ist, denen die Moleküle entsprechen müssen, um aktiv zu sein.

2. Computergestütztes Verfahren zum Bereitstellen, Identifizieren und Beschreiben von Molekülen mit einem gewünschten Verhalten, insbesondere auf dem Gebiet der Pharmakologie, nach Anspruch 1, **dadurch gekennzeichnet, dass** es im Wesentlichen die folgenden aufeinanderfolgenden Schritte umfasst:
1) das Erstellen einer Lernbasis, ausgehend von einer Gruppe bekannter Molekülstrukturen, die einerseits aus aktiven Molekülstrukturen, die die gewünschte Aktivität aufweisen, und andererseits aus bekannten inaktiven Molekülstrukturen besteht, denen diese Aktivität fehlt oder die schwache Aktivität aufweisen, indem entsprechende Deskriptoren verwendet werden;
2) das von der Lernbasis ausgehende Schaffen einer kombinatorischen Explosion von Molekülen;
3) das Durchführen einer Sichtung der so erzeugten Moleküle auf Basis der Anreicherung in Bezug auf die molekulare Vielfalt, die jedes Molekül gegenüber ausgewählten Deskriptoren zeigt;
4) das Filtern der so beibehaltenen Molkeüle mit statischen Filtern, die für Beschränkungen der strukturellen, physikochemischen und molekularen Konformationsschwankung repräsentativ sind, denen die Moleküle entsprechen müssen, um aktiv zu sein, und gegebenenfalls das Synthetisieren und Testen der selektierten Moleküle;
5) das Filtern der vielversprechendsten so ausgewählten Moleküle mit einem dynamischen Filter, der für Beschränkungen der Konformationsschwankung repräsentativ ist, denen die Moleküle entsprechen müssen, um aktiv zu sein;
6) das Synthetisieren und Testen der so selektierten Moleküle.

3. Computergestütztes Verfahren zum Bereitstellen, Identifizieren und Beschreiben von Molekülen mit einem gewünschten Verhalten, insbesondere auf dem Gebiet der Pharmakologie, nach Anspruch 2, **dadurch gekennzeichnet, dass**, wenn das erhoffte Ergebnis nicht oder nur teilweise erzielt wird, die Schritte 3) bis 6) mit modifizierten Filtern wiederholt werden.

4. Computergestütztes Verfahren zum Bereitstellen, Identifizieren und Beschreiben von Molekülen mit einem gewünschten Verhalten, insbesondere auf dem Gebiet der Pharmakologie, nach Anspruch 2 oder 3, **dadurch gekennzeichnet, dass** das dynamische Filtern mithilfe einer Software durchgeführt wird, die es ermöglicht, die Konformationsräume beliebiger Moleküle ausgehend von Bahnen der Moleküldynamik zu charakterisieren.

5. Computergestütztes Verfahren zum Bereitstellen, Identifizieren und Beschreiben von Molekülen mit einem gewünschten Verhalten, insbesondere auf dem Gebiet der Pharmakologie, nach einem der vorangegangenen Ansprüche, **dadurch gekennzeichnet, dass** beim dynamischen Filtern der Deskriptor in einer Form eingesetzt wird, die vom 3D-Autokorrelationsvektor (3D-ACV) bereitgestellt wird.

6. Computergestütztes Verfahren zum Bereitstellen, Identifizieren und Beschreiben von Molekülen mit einem gewünschten Verhalten, insbesondere auf dem Gebiet der Pharmakologie, nach Anspruch 5, **dadurch gekennzeichnet, dass** das dynamische Filtern darin besteht, für jede Reihe von aus der Moleküldynamik (MD) erhaltenen Konformationen einen 3D-ACV-Satz zu berechnen, der anschließend behandelt wird, indem multivariabte Statistiken in drei Hauptschritten (i bis iii) eingesetzt werden:
(i) für eine bestimmte Konformation des untersuchten Moleküls wird der entsprechende 3D-ACV-Deskriptor durch Bestimmung der Entfernungen zwischen allen Atompaaren und der Verteilung dieser Entfernungen berechnet, wodurch ein Vektor gebildet wird, dessen jeweiliger "Topf" ("Bin") die Summe der Atompaare in einem spezifischen Bereich interatomarer Abstände ist;
(ii) für jede Konformation wird ein Speicher berechnet, wobei das 3D-ACV-Vielfache dem "Flug" entspricht;
(iii) eine Analyse der Hauptkomponenten (PCA) wird an jedem dieser 3C-ACV-Vielfachen vorgenommen.

7. Computergestütztes Verfahren zum Bereitstellen, Identifizieren und Beschreiben von Molekülen mit einem gewünschten Verhalten, insbesondere auf dem Gebiet der Pharmakologie, nach Anspruch 4, **dadurch gekennzeichnet, dass** beim statischen Filter physikochemische oder topologische Deskriptoren zum Einsatz kommen, die zumindest zum Teil aus Folgenden ausgewählt sind: Molmasse, Ellipsoidvolumen, Molekülvolumen, Molenbruch, Lipophilie (LogP), kappa 1, kappa 2, kappa 3, kappa alpha 1, kappa alpha 2, kappa alpha 3, Flexibilität, Kier-chi V4, Randic-Index, Balaban-Index, Wienerlndex, E-Zustands-Summe, Dipolmoment, Anzahl der C-Atome, Anzahl der O-Atome, Anzahl der N-Atome, Anzahl der H-Atome, Gesamtanzahl der Atome, Anzahl der Methylgruppen, Anzahl der Ethylgruppen, Anzahl der Aminogruppen und Anzahl der Hydroxygruppen

## Claims

1. Computer-assisted method for providing, identifying and describing molecules having a required behaviour, in particular in the field of pharmacy, **characterized in that** it comprises:
1) providing a learning base from a family of known molecular structures that is composed on the one hand of active molecular structures exhibiting the required activity and on the other hand of inactive molecular structures known to be devoid of this activity, or as exhibiting weak activity, by using appropriate descriptors;
2) generating static filters representative of the intervals of the structural, physico-chemical and molecular values with which the molecules must comply in order to be active from the learning base;
3) performing a screening of a combinatorial bank on account of these static filters;
4) subjecting the most promising molecules thus selected to a dynamic filter representative of the constraints of conformational variation that the molecules must observe in order to be active.

2. Computer-assisted method for providing, identifying and describing molecules having a required behaviour, in particular in the field of pharmacy, according to Claim 1, **characterized in that** it essentially comprises the success steps consisting in:
1) producing a learning base from a family of known molecular structures that is composed on the one hand of active molecular structures exhibiting the required activity and on the other hand of inactive molecular structures known to be devoid of this activity, or as exhibiting weak activity, by using appropriate descriptors;
2) generating a combinatorial explosion of molecules from the learning base;
3) performing a screening of the molecules thus generated on the basis of the enrichment, in terms of molecular diversity, afforded by each molecule with regard to the chosen descriptors;
4) subjecting the molecules thus adopted to static filters representative of the constraints of structural, physico-chemical and molecular variations with which the molecules must comply in order to be active and, possibly, synthesizing and testing the selected molecules;
5) subjecting the most promising molecules thus selected to a dynamic filter representative of the constraints of conformational variation that the molecules must observe in order to be active;
6) synthesizing and testing the molecules thus selected.

3. Computer-assisted method for providing, identifying and describing molecules having a required behaviour, in particular in the field of pharmacy, according to Claim 2, **characterized in that**, if the anticipated result is not obtained, or if it is obtained only partially, steps 3) to 6) are repeated with modified filters.

4. Computer-assisted method for providing, identifying and describing molecules having a required behaviour, in particular in the field of pharmacy, according to Claim 2 or 3, **characterized in that** the dynamic filtering can be performed by means of software that makes it possible to characterize the conformational spaces of any molecules from molecular-dynamics trajectories.

5. Computer-assisted method for providing, identifying and describing molecules having a required behaviour, in particular in the field of pharmacy, according to any one of the preceding claims, **characterized in that** the dynamic filtering implements the shape descriptor consisting of the 3D autocorrelation vector (3D-ACV).

6. Computer-assisted method for providing, identifying and describing molecules having a required behaviour, in particular in the field of pharmacy, according to Claim 5, **characterized in that** the dynamic filtering consists in calculating a set of 3D-ACVs for each series of conformations obtained by molecular dynamics (MD) and then processed using multivariate statistics in three main steps (i to iii):
(i) for a given conformation of the molecule studied, the corresponding 3D-ACV descriptor is calculated by determining the distances between all the pairs of atoms and the distribution of these distances forming a vector, each "bin" of which is the sum of pairs of atoms in a specific range of interatomic separation;
(ii) the corresponding multiple 3D-ACV is calculated and stored for each conformation "on the fly";
(iii) a principal components analysis (PCA) of each of these multiple 3C-ACVs is carried out.

7. Computer-assisted method for providing, identifying and describing molecules having a required behaviour, in particular in the field of pharmacy, according to Claim 4, **characterized in that** the static filter implements physico-chemical and topological descriptors, at least some of which are chosen from among molar mass, ellipsoidal volume, molecular volume, molar refractivity, lipophilia (LogP), kappa 1, kappa 2, kappa 3, kappa alpha 1, kappa alpha 2, kappa alpha 3, flexibility, kier chi V4, randic index, balaban index, wiener index, state sum E, dipolar moment, number of C atoms, number of O atoms, number of N atoms, number of H atoms, total number of atoms, number of methyl groups, number of ethyl groups, number of amino groups and number of hydroxy groups.
